# EUROPEAN PATENT APPLICATION

(11) **EP 3 384 855 A1**
(43) Date of publication of application: **10.10.2018**
(21) Application number: 17164716.7
(22) Date of filing: 04.04.2017
(51) Int. Cl.: A61B 10/00, A61B 5/00, A61B 5/103

(54) **UNOBTRUSIVE MENSTRUAL PHASE DETERMINING SYSTEM**

(71) Applicant: Koninklijke Philips N.V., 5656 AE Eindhoven (NL)
(72) Inventor: MENA BENITO, Maria Estrella, 5656 AE Eindhoven (NL); HUIJBREGTS, Laurentia Johanna, 5656 AE Eindhoven (NL); AARTS, Ronaldus Maria, 5656 AE Eindhoven (NL)
(74) Representative: de Haan, Poul Erik

(57) **Abstract**

An unobtrusive system (100) is designed to provide personalized and real-time information about a woman's menstrual cycle and the different phases thereof. The system (100) comprises an image detecting unit (10) that is arranged and configured to detect an image of at least a portion of a woman's face (101) and to output a detection signal representative of the image; a processing unit (20) that is arranged and configured to receive the detection signal from the image detecting unit (10) and to process the detection signal according to a preset routine in order to generate an information signal representative of information relating to the subject's menstrual phase; and a communication unit (31, 32) that is configured to generate output comprising the information relating to the subject's menstrual phase in a humanly processable form on the basis of the information signal.

## Description

### FIELD OF THE INVENTION

The invention relates to an unobtrusive menstrual phase determining system, i.e. a system that is configured to determine a phase of a subject's menstrual cycle in an unobtrusive fashion.

The invention furthermore relates to an assembly of a mirror and an unobtrusive menstrual phase determining system.

### BACKGROUND OF THE INVENTION

The menstrual cycle is the regular natural change that occurs in the female reproductive system that makes pregnancy possible. It is a known fact that the menstrual cycle is governed by hormonal changes. Each cycle can be divided into three phases based on events in the ovary, i.e. the ovarian cycle, or events in the uterus, i.e. the uterine cycle. The ovarian cycle involves a follicular phase, ovulation, and a luteal phase, whereas the uterine cycle involves menstruation, a proliferative phase, and a secretory phase.

Normally, women of reproductive age have their menstrual cycles each month. A regular menstrual cycle takes place once every 28 to 31 days, covering a period that starts at the first day of menstrual bleeding and lasts until the first day of the next menstrual bleeding, and the menstrual bleeding lasts for approximately 3 to 7 days. It is a commonly known fact that many women have irregular periods, i.e. that the length of the cycle may vary from month to month. Also, some women have relatively short cycles, lasting only 22 days, for example, and some women have relatively long cycles, lasting up to 36 days, for example.

For various reasons, it may be important to women to keep track of the phase in their menstrual cycle. For example, women suffering from premenstrual syndrome (PMS) may want to schedule important meetings or trips outside the period with severe PMS symptoms. In view of birth control or a desire to get pregnant, women may want to know if they are in a fertile phase in their menstrual cycle, or not. In general, it is practical and convenient for women to know when their menstruation is going to take place, so that they may know if they should take sanitary napkins and/or tampons with them when leaving the house, or if they can go swimming or participate in another physical activity.

Many women try to determine the phase in the menstrual cycle by marking the starting day of their menstrual bleeding on a calendar and counting how many days have passed since then at a certain point. However, this way of doing is not practical and not reliable, especially for women having irregular periods.

Various techniques for predicting ovulation are currently available in the market. However, the known methods and devices are disadvantageous in that they require discipline, are obtrusive and/or may make a woman feel embarrassed. For example, in a method relying on temperature measurements, a user must wake up at the same time every day to take her temperature. Furthermore, ovulation sticks for detecting hormone levels in urine require bothersome and potentially unhygienic actions from a user. Wearing a watch or bracelet for indicating ovulation is an improvement, but shows to other people that a woman is trying to get pregnant.

When it comes to birth control, special calendars and fertility apps are available that are intended to help with timing sexual abstinence to coincide with the fertile phase in a woman's menstrual cycle. However, trying to realize birth control in such a way is not very safe, in view of the fact that even though the mechanism of menstruation is repetitive, it often has an irregular character.

Management of women suffering from PMS requires detailed history taking, prospective diary recording of symptoms during several months.

It follows from the foregoing that there is a need for a simple, unobtrusive and inconspicuous way of generating information about the phase in a woman's menstrual cycle in a personal way. In this respect, it is noted that an unobtrusive ovulation tracking system is known from WO 2015/150434 A1. The system comprises a sensor for obtaining a subject's heart signal, a processing unit configured to determine the subject's heart rate from the heart signal, and an evaluation unit configured to analyze the heart rate to predict likelihood of ovulation. The processing unit is further configured to extract heart rate variability features from the heart signal, and the evaluation unit is further configured to predict likelihood of ovulation based on the heart rate variability features. Thus, according to WO 2015/150434 A1, the likelihood of ovulation is predicted based on the heart rate. Advantageously, the assessment is an analysis of the heart rate over time, knowing that during ovulation the resting heart rate is significantly higher than during other times of the menstrual cycle. In view thereof, the evaluation unit is configured to detect periods of relatively high heart rate.

### SUMMARY OF THE INVENTION

The unobtrusive system known from WO 2015/150434 A1 is only suitable for indicating ovulation and cannot be used for indicating other phases of the menstrual cycle. It is an object of the invention to provide an unobtrusive system that is more sophisticated in that it can be used for reliably indicating a fertile window, predicting menstruation, and/or indicating if a woman is in the follicular phase, the ovulatory phase, the luteal phase or the bleeding phase of her menstrual cycle.

The invention provides an unobtrusive menstrual phase determining system, comprising: an image detecting unit that is arranged and configured to detect an image of at least a portion of a subject's face and to output a detection signal representative of the image; a processing unit that is arranged and configured to receive the detection signal from the image detecting unit and to process the detection signal according to a preset routine in order to generate an information signal representative of information relating to the phase in the subject's menstrual cycle, using components of the detection signal related to at least one feature that fluctuates with the menstrual cycle in processing the detection signal; and a communication unit that is arranged and configured to generate output comprising the information relating to the phase in the subject's menstrual cycle in a humanly processable form by receiving the information signal from the processing unit and using the information signal in generating the output.

Different from the system known from WO 2015/150434 A1, the system according to the invention relies on detecting an image of at least a portion of a subject's face and analyzing the image so as to determine information relating to the phase in the subject's menstrual cycle. Detecting an image allows for unobtrusive monitoring and tracking of daily fluctuations in facial profile and characteristics, whereas the system known from WO 2015/150434 A1 is only adapted to measure/monitor heart rate, heart rate variability, and changes thereof.

The image detecting unit of the system according to the invention may comprise at least one camera. Preferably, the at least one camera is integrated in a mirror such as a bathroom mirror. In such a case, the mirror may be a normal reflective mirror with one or more cameras attached to it, or a two-way mirror with a camera arranged behind it. Alternatively, the at least one camera may be integrated in a computer screen, for example, assuming that the subject to be monitored can be expected to sit in front of the computer screen on a regular/daily basis.

The processing unit of the system is configured to analyze the images from the image detecting unit so as to determine the phase in the subject's menstrual cycle. The analysis is done by following a preset routine that is particularly designed to involve at least one feature that can be derived from the detected image and that is known to fluctuate with the menstrual cycle.

Information relating to the phase in the subject's menstrual cycle may be as simple as an indication as to whether a subject is in her fertile window, or not. According to another practical option, which could be combined with such relatively simple indication, the information may be a number of days, for example, a number of days left until ovulation or menstruation.

The communication unit of the system serves for communicating the information relating to the phase in the subject's menstrual cycle, as determined by the processing unit, to a user of the communication unit, who may be the subject herself and/or a another person such as a caregiver. Outputting the information may be done through an app on the user's phone, for example, or on a suitable display. In a general sense, the communication unit is adapted to generate output in a humanly processable form, i.e. in a form that can be detected by at least one of the human senses and processed in the human brain so as to make a human aware of the output of the communication unit. Practical examples of output in a humanly processable form include output as can be detected by human vision and output as can be detected by human hearing. The processing unit may be configured to transmit the information signal as a wireless signal, wherein there is no need for the processing unit and the communication unit to be physically connected in any way. The internet may be used for conveying the information signal to any person to whom the information about the phase in the subject's menstrual cycle may be of interest.

According to a practical option as mentioned in the foregoing, the system according to the invention may be associated with a mirror, wherein at least the image detecting unit of the system may be integrated in the mirror, in which case the mirror may be used for displaying the information. Instead of or in addition to visually displaying the information, it is possible to provide audio feedback or even tactile feedback. For example, it may be so that a watch or a phone is made to vibrate as soon as it is determined that a subject has reached her fertile window.

The routine that is followed by the processing unit during operation of the system according to the invention may be preprogrammed in the processing unit. More than one routine may be available in the system, and the system may be equipped with a user interface to provide a user with the possibility of setting a routine as desired. Following the routine may involve applying preprogrammed algorithms, using look-up tables, etc. In any case, in the context of the invention, the routine is aimed at analyzing input about one or more features of a subject's face, especially features that are known to fluctuate with the menstrual cycle.

It may be so that the processing unit is adapted to determine an actual phase in the subject's menstrual cycle by comparing actual detected features to preset reference values or preset ranges of values, but it may especially be practical if the processing unit is configured to make a comparison of actual detected features and previously detected features in order to find a development of the features over time and derive relevant information from such development. In particular, it is possible for the processing unit to comprise a memory that is configured to store detection input derived from at least one detection signal, and to be configured to make a comparison between detection input derived from an actual detection signal and detection input derived from at least one earlier detection signal, as stored in the memory, and to use an outcome of the comparison in the process of generating the information signal.

As mentioned in the foregoing, according to the invention, the processing unit is configured to use components of the detection signal related to at least one feature that fluctuates with the menstrual cycle in processing the detection signal when making the analysis for obtaining information relating to the phase in the subject's menstrual cycle. In particular, the at least one feature may be at least one feature of the subject's face. An example of such a feature is face contour. More in particular, the at least one feature may be at least one feature of the subject's facial skin. Even more in particular, the at least one feature may be at least one of skin pigmentation, skin color, skin shininess, pore size, hydration level, occurrence of pimples and occurrence of skin irritation.

The fact is that hormonal cycles influence a woman's body over time. It is known that peripheral skin circulation varies significantly within a menstrual cycle, and also that skin characteristics change in response to daily fluctuations within the menstrual cycle. For instance, a pigmentation of the skin varying in intensity in a constant relation to the phases in the menstrual cycle has been recognized by clinicians for many years. The pattern of this variation and its occurrence in normal young women has been demonstrated by a simple survey technique. The occurrence of the skin condition appears to be related to the sensitivity of the skin to ultraviolet light; darkening of the skin always occurring in the premenstrual week. There is also variation in redness of the skin throughout the menstrual cycle, which is however not easily visibly by eye.

During the menstrual phase, estrogen and progesterone levels both fall, though secretions of the former start to gradually rise halfway through the phase. The body temperature falls during menstruation, which slows the blood circulation and the metabolism leaving the skin with a dullish appearance, less red than in the other phases. Sebum secretions decrease and the moisture-retention functions of the skin deteriorate. These changes dry the skin and promote chapping. Overall, the skin becomes very sensitive.

During the follicular phase, i.e. the phase following menstruation, secretions of estrogen increase. Estrogen encourages the generation of collagen. The general condition of the mind, body and skin improves. The complexion and skin circulation also improve, and a good balance of moisture and oils in the skin is maintained. The redness of the skin increases during this phase.

During the ovulation phase, as estrogen levels begin to rise, the hormone attaches to cell receptors in the skin and promotes the production of glycosaminoglycans like hyaluronic acid in the skin, which allows the skin to remain hydrated by attracting water molecules, and which upholds the skin structure. Thus, the skin looks its feminine best when women are most fertile. Estrogen peaks and suppresses sebum production just before ovulation, so the skin is less oily and pores are at their tiniest. In this phase, there is higher contrast between facial skin color and eye/lip color.

During the luteal phase, the body secretes high levels of progesterone. The risk of skin problems is highest about then. Secretions of sebum rise together with the body temperature, which creates a skin environment favorable for acne and other minor problems. The skin also tends to dull, and pigments in the skin darken. From mid-cycle on, the skin steadily reddens because of an increase in blood flow that peaks in the day or in two days preceding menstruation.

It follows from the foregoing that skin redness is a preferable feature for use in the process of analyzing the input obtained from the detection of an image of at least a portion of a subject's face. Within the framework of the invention, one or more other features may be used in addition or as an alternative. For example, it is possible to use the system according to the invention to monitor face contour related changes. Changes in face contour are related to fluid retention (edema) and also to physical changes during the menstrual cycle, so that face contour can be denoted as being a feature that fluctuates with the menstrual cycle. In such a case, the processing unit of the system according to the invention is configured to use components from the detection signal related to the contour of the subject's face in processing the detection signal. In practice, the whole facial image as detected by the image detecting unit may be interpreted in the process of determining the information signal.

In an advantageous embodiment, the system according to the invention is adapted to realize face recognition. In other words, it is advantageous if the system is configured to automatically recognize an image as being an image of a face, and if so, to assess whether an actual image of a face is an image of a known subject's face. The latter is especially convenient if the image detecting unit is associated with a mirror that is not only used by the subject but also by other persons. In terms of characteristics of the system according to the invention, face recognition may be realized when the system comprises a user interface that is arranged and configured to receive input from a user and to output a user signal representative of the input, including a confirmation as to whether a detection signal is related to a relevant subject whose phase in the menstrual cycle is to be determined, when the processing unit comprises a memory that is configured to store information related to at least one feature of the relevant subject's face, and when the processing unit is additionally configured to process the detection signal according to a face recognition routine that involves comparing information related to at least one feature of an actual subject's face to information related to at least one feature of a relevant subject's face, as stored in the memory.

Optionally, the system is designed to realize a learning phase as a measure that really enables the system to be a personalized menstrual cycle tracking system. To that end, it may be so that the system comprises a user interface that is arranged and configured to receive input from a user and to output a user signal representative of the input, including an indication of an actual phase in the subject's menstrual cycle, wherein the processing unit is configured to be operated in one of a learning mode and a normal operative mode, and wherein the learning mode involves storing combinations of information related to at least one feature of the relevant subject's face and a phase in the menstrual cycle, as indicated by the user of the user interface.

In order for the determination of the information relating to a phase in the subject's menstrual cycle to be accurate, it is an advantageous option to take into account circumstantial factors that may influence the at least one feature to be assessed besides factors that are directly related to the menstrual cycle. To that end, the system according to the invention may comprise at least one device that is configured to output a circumstantial signal representative of an actual value of a circumstantial factor, wherein the processing unit may be arranged and configured to receive the circumstantial signal from the device and to use the circumstantial signal in processing the detection signal, particularly to use the circumstantial signal for making corrections aimed at determining what the at least one feature to be assessed would be if there would be no influence of circumstantial factors. To mention a few of many practical possibilities existing within the framework of the invention, the at least one device may be at least one of a clock that is configured to output a circumstantial signal representative of an actual time, an activity tracker that is arranged and configured to sense a subject's activity and to output a circumstantial signal representative of the subject's activity, a thermometer that is arranged and configured to sense an ambient temperature and to output a circumstantial signal representative of the ambient temperature, and a moisture sensor that is arranged and configured to sense an ambient moisture level and to output a circumstantial signal representative of the ambient moisture level.

Various studies have demonstrated that a correlation exists between the phase in the menstrual cycle and vital signs such as respiratory rate and heart rate. For example, it is known that for women with PMS, respiratory rate and heart rate are significantly increased in the luteal phase when compared to the follicular phase and the menstrual phase. Also, it is known that in general, so also for women not suffering from PMS, respiratory rate and heart rate increase throughout the cycle, and also heart rate variability decreases. In respect of heart rate variability, it has been shown how several features related to heart rate variability during sleep are different for the late-luteal phase compared to the follicular phase, both for women with and without PMS, and it also has been shown that a larger stroke volume is obtained in the luteal phase compared to the follicular phase.

The system according to the invention may be adapted to apply knowledge of known correlations between the phase in the menstrual cycle and vital signs, and to involve actual information about one or more vital signs in the process of determining the information relating to a phase in the subject's menstrual cycle. Hence, the system may comprise at least one device that is arranged and configured to sense a subject's vital sign and to output a vital sign signal representative of a value of a subject's vital sign, wherein the processing unit may be arranged and configured to receive the vital sign signal from the device and to use the vital sign signal in processing the detection signal. In such a case, it may be practical if the processing unit is configured to process the vital sign signal according to a preset routine in order to generate a secondary information signal representative of information relating to the actual value of the subject's vital sign, and if the communication unit is configured to generate output comprising the information relating to the actual value of the subject's vital sign in a humanly processable form by receiving the secondary information signal from the processing unit and using the secondary information signal in generating the output, so that the system may not only be used for providing information about a phase in the subject's menstrual cycle, but also for providing information about one or more vital signs of the subject.

As mentioned in the foregoing, the image detecting unit may comprise at least one camera. For example, the system may be equipped with at least one camera that is arranged and configured to measure a PPG (photoplethysmographic) signal from an area of the subject's facial skin at various wavelengths, and wherein the processing unit is configured to generate a PPG imaging map of spatial distribution of at least one of the amplitude, the phase and the shape of the PPG signal.

In general, camera-based PPG provides non-contact measurement of a pulse signal by analyzing subtle changes of skin color, which are not visible to the human eye, and which correspond to changes in blood volume. In the system according to the invention, the camera may be used to extract a pulse signal from small areas of skin, thus generating PPG imaging, i.e. a spatial map of the pulse amplitude, the pulse phase and the pulse shape. Such a map is useful in the context of the invention on the basis of the known fact that the amplitude or pulsatility of a PPG signal can be influenced by local skin properties. For instance, changes in the skin features, i.e. deterioration of skin, apparition of pimples, involve an increase of the amplitude of a PPG signal that is extracted locally from under the skin surface. Spatial distribution (imaging) of PPG amplitudes and phase differences contains valuable information about local microcirculation of skin. Furthermore, camera-based PPG allows to measure a heartbeat signal by analyzing temporal modulation of light reflected from a skin tissue. The reflected light, which may be either ambient light or dedicated light, is modulated due to changes of arterial blood volumes with every pulse.

The above-described and other aspects of the invention will be apparent from and elucidated with reference to the following detailed description of an unobtrusive system for tracking phases in the menstrual cycle of a subject.

### BRIEF DESCRIPTION OF THE DRAWINGS

The invention will now be explained in greater detail with reference to the figures, in which:
Figure 1 diagrammatically shows a number of components of an embodiment of an unobtrusive menstrual phase determining system according to the invention;
Figure 2 is a general block scheme of elements of the unobtrusive menstrual phase determining system; and
Figure 3 illustrates a preferred embodiment of one of the elements indicated in figure 2.

### DETAILED DESCRIPTION OF EMBODIMENTS

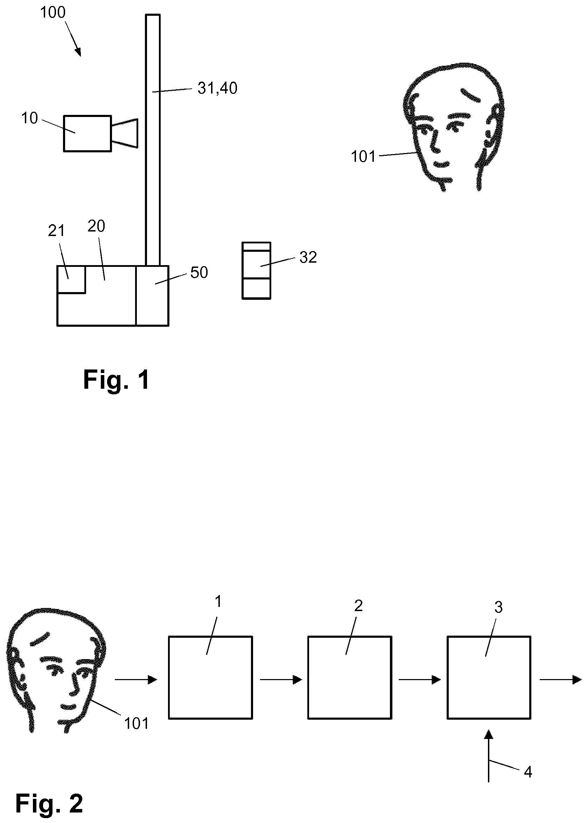

Figures 1-3 relate to an embodiment of an unobtrusive menstrual phase determining system according to the invention. In the figures, the system is indicated by means of reference numeral 100. The system 100 is designed to be used for obtaining information about the actual phase in the menstrual cycle of a subject, i.e. of a woman who is to be investigated by means of the system 100, wherein the level of the information may range from general to specified, whatever is desired. For example, it may be sufficient for a woman to know whether she is in her fertile window, or not, or to know when to expect menstruation, whereas a professional caregiver of a woman may want to be informed whether the woman is in the menstrual phase, the follicular phase, the ovulation phase or the luteal phase. The system 100 may be denoted as being an electronic system 100 comprising a number of electronic components that are arranged and configured to realize the functionality as desired.

In general, the embodiment of the system 100 according to the invention as illustrated in figure 1 comprises a camera 10, a processor 20, a first display device 31 and a second display device 32, the camera 10 being a practical example of an image detecting unit of the system 100, the processor 20 being a practical example of a processing unit of the system 100, and the display devices 31, 32 being a practical example of a communication unit of the system 100. The various units of the system 100 may be interconnected in any suitable way, particularly through wire or wireless. In the latter case, communication from one unit to another can be established through any suitable wireless technique, including through Bluetooth and through the internet. In any case, the various units of the system are arranged and configured to communicate on the basis of an appropriate transfer of signals, particularly electronic signals. In this respect, it is noted that the image detecting unit is configured to output a detection signal that is representative of a detected image, the processing unit is arranged and configured to receive the detection signal from the image detecting unit, and is configured to output an information signal that is representative of information about the phase in a subject's menstrual cycle, and the communication unit is arranged and configured to receive the information signal from the processing unit.

In the shown example, the system 100 is combined with a mirror 40, particularly a mirror that is designed to function as the first display device 31. In this case, the mirror 40 is a two-way mirror with the camera 10 arranged behind it, but that does not alter the fact that it is also possible to use a normal reflective mirror with the camera 10 attached to it at a position for detecting at least a portion of a face of a person standing in front of the mirror. Furthermore, in the shown example, the second display device 32 is a mobile phone. Within the framework of the invention, any suitable type of device for conveying information to a human being may be applied in the system 1, wherein it is noted that a computer screen in communication with a computer is another example out of various possibilities.

The camera 10 is used to capture images of a woman's face 101 as caught by the mirror 40 and may be a normal RGB camera. Instead of or in addition to an RGB camera, a 3D camera and/or a thermal camera could be used. The processor 20 is configured to analyze the images from the camera 10 on features that fluctuate with the menstrual cycle, and to thereby determine the phase in the menstrual cycle. A preferable feature that may be used in the process is skin redness, but one or more other features may be used as well. Skin features that are of importance, i.e. that fluctuate with the menstrual cycle and that can be measured with a camera include redness, dryness/shininess, pore size, hydration level, occurrence of pimples and occurrence of skin irritation such as eczema. Optionally, the processor 20 may be configured to not only analyze the camera images for one or more features of a woman's facial skin, but also for one or more other features that are also known to fluctuate with the menstrual cycle such as face contour, blood oxygenation, heart rate and heart rate variability, respiration rate and skin temperature. In this way, reliability of an estimation of the ovulation time and an assessment of PMS may be further enhanced. Assessing face contour can be done by using a 2D camera or a 3D camera, and may particularly be aimed at signaling edema. Heart rate and heart rate variability may be assessed by using a specific vital signs camera. Such a camera may be a PPG camera, in which case the PPG amplitude may be used to determine one or more cardiac characteristics. Skin temperature may be determined by using a thermal/infrared camera.

For the sake of completeness, in respect of the option of applying PPG techniques, the following is noted. In the system 100 according to the invention, a camera or a set of cameras for measuring PPG signals from a large skin area at various wavelengths, either in a visible or in a near-infrared spectrum, may be applied. In such a case, the processor 20 is used to analyze PPG pulsatility at various wavelengths in the visible and the nearly-infrared spectrum, per small block of pixels. As a result, a PPG imaging map of spatial distribution of PPG amplitude is generated. An advantage of using PPG imaging over using visible skin color detection is that a detection of skin areas following from detection of high PPG pulsatility provides earlier indication of skin features in a contactless manner. In the process, dedicated or ambient illumination may be used as a source of light. In fact, it is sufficient to have a single wavelength of illumination, but having additional wavelengths is preferred so as to improve robustness and accuracy.

In the shown example, the system 100 according to the invention comprises a user interface 50, so that a user may provide input to the processor 20 for the purpose of providing information to the processor 20 and/or determining operational factors of the processor 20. In respect of the latter option, it is noted that the processor 20 may be designed to function in one of at least two modes, namely a normal operative mode and a learning mode. In the learning mode, a woman using the system 100 (or another person receiving information from the woman) enters one or more of known phases of her menstrual cycle in the system 100 through the user interface 50 for one or more months. The processor 20 has a memory 21 to store data related to input from both the user interface 50 and the camera 10, and is programmed to correlate the input about the phases of the menstrual cycle with the camera images it has captured in this time period. Examples of known phases are the first day of menstruation, and also ovulation (to be determined by using a conventional urine stick or otherwise).

Optionally, the system 100 comprises an activity tracker so as to provide the processor 20 with input that may cause the processor 20 to exclude images captured just after high activity, which would increase the temperature and redness of the skin. Also, data from a thermometer could be included for a measurement of the ambient temperature, which may be relevant in view of the fact that redness and skin temperature changes may be related to a change of ambient temperature. Especially in case the room where the system 100 detects the images of a woman's face 101 is a bathroom, it could be practical to use a moisture sensor for detecting whether the woman has just taken a bath/shower, which might also influence redness and skin temperature and thereby cause the input to the system 100 to be non-suitable for processing. Also, input from a moisture sensor could be used for correcting for the influence of dry weather or dry circumstances in a room on the moisture level of the skin. A clock could be used to ensure that only images captured at approximately the same moment of the day, for example, just after getting up or just before going to bed, are taken into account. In general, one or more appropriate devices may be used with the system 100 for the purpose of obtaining information about circumstantial influences, so that it is possible to make corrections for those influences, and to thereby increase reliability of the estimation of the changes and/or fluctuations of facial features.

It follows from the foregoing that the system 100 according to the invention is capable of providing information on the basis of which it can be known on which days the events of a menstrual cycle will occur on a predictable basis. The system 100 may particularly be configured to relate changes in facial features to phases in the menstrual cycle. The system 100 is unobtrusive and involves notable advantages, as it is suitable to be used in management of PMS symptoms in a personal and optimal way, and for the purpose of determining a fertile window of a woman, which may be useful in respect of both birth control purposes and planned parenthood purposes.

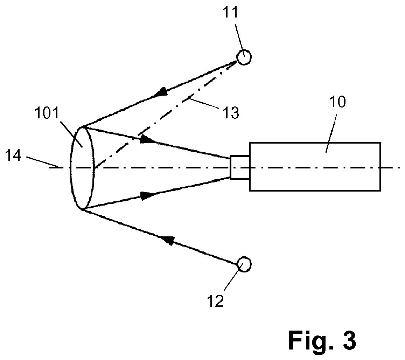

Figure 2 shows a block scheme of elements of the embodiment of the system 100 according to the invention, which embodiment is one of a plurality of possible practical realizations of the system 100. In the block scheme, the following elements are successively indicated, besides a woman's face 101: (1) image detection 1 by means of the camera 10 and associated lighting, as will be explained further below with reference to figure 3, (2) color correction 2 as one of the functionalities of the processor 20 to make the analysis to be performed by the processor 20 robust against changes in ambient lighting, (3) trend detection 3 and (4) aggregation 4 of other inputs such as results of a white blood cell count, a red blood cell count, a cortisol level determination, a core body temperature measurement, etc.

When the system 100 is operated, images of facial skin are first captured using the camera 10. The images are calibrated to ensure consistency in feature extraction and analysis. The system 100 may particularly be configured to obtain the facial images by automatically extracting four blocks, one on the forehead, one on the nose, one below the left eye and one below the right eye, and using the four blocks to represent a face. The processor 20 may be configured to combine a number of blocks for the purpose of constructing total facial tissue images. In figure 3, it is illustrated how the camera 10 may be used in combination with fluorescent lamps 11, 12 for lighting the woman's face 101. It is practical if the lamps 11, 12 are arranged at a position that is in a plane 13 extending approximately at 45° with respect to a central basic plane 14 intersecting both the camera 10 and the face 101 at a central position.

The option of color correction is advantageous in view of the fact that color images produced by digital cameras are usually device-dependent, which implies that generated color information (usually presented in RGB color space) is usually dependent on the imaging characteristics of specific cameras. By using an appropriate correction scheme, the device-dependency is accounted for.

Operation of the system 100 according to the invention involves a face profile - baseline estimation as a first stage of operation, which, with reference to the various options described in the following, may involve the following: (i) operation of a 3D camera or a set of cameras, possibly integrated into a mirror 40, for obtaining information about the shape of a woman's face 101; (ii) registration of a profile of the woman's facial skin, which may involve operating a set of cameras for measuring a number of different skin features through measuring a PPG signal from the facial skin area; (iii) calculation of the images over both portions of the facial skin area and the whole of the facial skin area; (iv) introduction of personal parameters such as age and general racial type; and (v) operation of an infrared camera for measurements of temperature and microcirculation.

Operation of the system 100 furthermore involves day to day monitoring of facial features of a woman whose phase in the menstrual cycle is to be determined. The woman needs to be at a position for allowing the at least one camera 10 of the system 100 to detect images of the woman's face 101 as desired for a short time. The processor 20 analyzes the images for fluctuations in the facial features. Practical examples of the facial features include skin unevenness (eczema, pimples), skin color, shininess, pore size and hydration level.

It is practical and effective if the processor 20 is especially operated to perform a comparative analysis of an actual image and the baseline, wherein values of the measured differences are determined. As mentioned in the foregoing, the comparison is preferably done on the basis of measurement results that are obtained at approximately the same moment on each day, and preferably not after high activity or in another circumstance that may influence one or more of the facial features used in the analysis.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims or the equivalents thereof. While the invention has been illustrated and described in detail in the figures and the description, such illustration and description are to be considered illustrative or exemplary only, and not restrictive. The invention is not limited to the disclosed embodiments. The drawings are schematic, wherein details that are not required for understanding the invention may have been omitted, and not necessarily to scale.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

The term "comprise" as used in this text will be understood by a person skilled in the art as covering the term "consist of'. Hence, the term "comprise" may in respect of an embodiment mean "consist of", but may in another embodiment mean "contain/include at least the defined species and optionally one or more other species".

Summarizing, the system 100 according to the invention is designed to provide personalized and real-time information about a woman's menstrual cycle and the different phases thereof. In view thereof, the system 100 is suitable to be used for prediction and detection of ovulation time and/or fertility window, which can be used by women trying to get pregnant or wanting to avoid pregnancy. Furthermore, the system 100 is suitable to be used in a process of diagnosing a woman with PMS and for estimation of menstruation and/or PMS symptoms approaching.

## Claims

1. Unobtrusive menstrual phase determining system (100), comprising:
- an image detecting unit (10) that is arranged and configured to detect an image of at least a portion of a subject's face (101) and to output a detection signal representative of the image;
- a processing unit (20) that is arranged and configured to receive the detection signal from the image detecting unit (10) and to process the detection signal according to a preset routine in order to generate an information signal representative of information relating to the phase in the subject's menstrual cycle, using components of the detection signal related to at least one feature that fluctuates with the menstrual cycle in processing the detection signal; and
- a communication unit (31, 32) that is configured to generate output comprising the information relating to the phase in the subject's menstrual cycle in a humanly processable form by receiving the information signal from the processing unit (20) and using the information signal in generating the output.

2. System (100) according to claim 1, wherein the processing unit (20) comprises a memory (21) that is configured to store detection input derived from at least one detection signal, and wherein the processing unit (20) is configured to make a comparison between detection input derived from an actual detection signal and detection input derived from at least one earlier detection signal, as stored in the memory (21), and to use an outcome of the comparison in the process of generating the information signal.

3. System (100) according to claim 1 or 2, wherein the processing unit (20) is configured to use components from the detection signal related to at least one feature of the subject's face (101) in processing the detection signal.

4. System (100) according to claim 3, wherein the processing unit (20) is configured to use components from the detection signal related to face contour in processing the detection signal.

5. System (100) according to any of claims 1-3, wherein the processing unit (20) is configured to use components from the detection signal related to at least one feature of the subject's facial skin in processing the detection signal.

6. System (100) according to claim 5, wherein the processing unit (20) is configured to use components from the detection signal related to at least one of skin pigmentation, skin color, skin shininess, pore size, hydration level, occurrence of pimples and occurrence of skin irritation in processing the detection signal, and wherein optionally the processing unit (20) is configured to use components from the detection signal related to skin redness in processing the detection signal.

7. System (100) according to any of claims 1-6, comprising a user interface (50) that is arranged and configured to receive input from a user and to output a user signal representative of the input, including a confirmation as to whether a detection signal is related to a relevant subject whose phase in the menstrual cycle is to be determined, wherein the processing unit (20) comprises a memory (21) that is configured to store information related to at least one feature of the relevant subject's face (101), and wherein the processing unit (20) is additionally configured to process the detection signal according to a face recognition routine that involves comparing information related to at least one feature of an actual subject's face (101) to information related to at least one feature of a relevant subject's face (101), as stored in the memory (21).

8. System (100) according to any of claims 1-7, comprising a user interface (50) that is arranged and configured to receive input from a user and to output a user signal representative of the input, including an indication of an actual phase in the subject's menstrual cycle, wherein the processing unit (20) is configured to be operated in one of a learning mode and a normal operative mode, and wherein the learning mode involves storing combinations of information related to at least one feature of the relevant subject's face (101) and a phase in the menstrual cycle, as indicated by the user of the user interface (50).

9. System (100) according to any of claims 1-8, comprising at least one device that is configured to output a circumstantial signal representative of an actual value of a circumstantial factor, wherein the processing unit (20) is arranged and configured to receive the circumstantial signal from the device and to use the circumstantial signal in processing the detection signal.

10. System (100) according to claim 9, wherein the at least one device is at least one of a clock that is configured to output a circumstantial signal representative of an actual time, an activity tracker that is arranged and configured to sense a subject's activity and to output a circumstantial signal representative of the subject's activity, a thermometer that is arranged and configured to sense an ambient temperature and to output a circumstantial signal representative of the ambient temperature, and a moisture sensor that is arranged and configured to sense an ambient moisture level and to output a circumstantial signal representative of the ambient moisture level.

11. System (100) according to any of claims 1-10, comprising at least one device that is arranged and configured to sense a subject's vital sign and to output a vital sign signal representative of a value of a subject's vital sign, wherein the processing unit (20) is arranged and configured to receive the vital sign signal from the device and to use the vital sign signal in processing the detection signal.

12. System (100) according to claim 11, wherein the processing unit (20) is configured to process the vital sign signal according to a preset routine in order to generate a secondary information signal representative of information relating to the actual value of the subject's vital sign; and wherein the communication unit (31, 32) is configured to generate output comprising the information relating to the actual value of the subject's vital sign in a humanly processable form by receiving the secondary information signal from the processing unit (20) and using the secondary information signal in generating the output.

13. System (100) according to any of claims 1-12, wherein the image detecting unit comprises at least one camera (10) that is arranged and configured to measure a photoplethysmographic (PPG) signal from an area of the subject's facial skin at various wavelengths, and wherein the processing unit (20) is configured to generate a PPG imaging map of spatial distribution of at least one of the amplitude, the phase and the shape of the PPG signal.

14. System (100) according to any of claims 1-13, wherein the processing unit (20) is configured to transmit the information signal as a wireless signal.

15. Assembly of a mirror (40) and a system (100) according to any of claims 1-14, wherein at least the image detecting unit (10) of the system (100) is integrated in the mirror (40).
